(19) Europäisches Patentamt
European Patent Office
Office européen des brevets

(11) EP 2 650 384 A1

(12) **EUROPEAN PATENT APPLICATION**
published in accordance with Art. 153(4) EPC

(43) Date of publication:
**16.10.2013 Bulletin 2013/42**

(21) Application number: **11846544.2**

(22) Date of filing: **07.12.2011**

(51) Int Cl.:
***C13K 1/04*** (2006.01) ***B01D 61/02*** (2006.01)
***B01D 61/14*** (2006.01) ***B01D 61/58*** (2006.01)
***B01D 65/02*** (2006.01) ***B01D 69/08*** (2006.01)
***B01D 69/12*** (2006.01) ***B01D 71/56*** (2006.01)
***B09B 3/00*** (2006.01)

(86) International application number:
**PCT/JP2011/078248**

(87) International publication number:
**WO 2012/077697 (14.06.2012 Gazette 2012/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.12.2010 JP 2010274329**
**10.12.2010 JP 2010275408**

(71) Applicant: **Toray Industries, Inc.**
**Tokyo 103-8666 (JP)**

(72) Inventors:
- **KANAMORI, Satoko**
  **Otsu-shi, Shiga**
  **5208558 (JP)**
- **HANAKAWA, Masayuki**
  **Otsu-shi,**
  **Shiga 5208558 (JP)**
- **KURIHARA, Hiroyuki**
  **Otsu-shi,**
  **Shiga 5208558 (JP)**
- **TAKEUCHI, Norihiro**
  **Otsu-shi,**
  **Shiga 5208558 (JP)**
- **MINAMINO, Atsushi**
  **Otsu-shi,**
  **Shiga 5208558 (JP)**

(74) Representative: **Hoefer & Partner**
**Pilgersheimer Straße 20**
**81543 München (DE)**

# (54) METHOD FOR PRODUCING CONCENTRATED AQUEOUS SUGAR SOLUTION

(57) Provided is a method for producing a concentrated aqueous sugar solution using a cellulose-containing biomass as a raw material, the method comprising the steps of:
(1) hydrolyzing a cellulose-containing biomass to produce an aqueous sugar solution;
(2) filtering the aqueous sugar solution obtained in (1) through a microfiltration membrane and/or an ultrafiltration membrane, and recovering an aqueous sugar solution from the permeate side; and
(3) filtering the aqueous sugar solution obtained in (2) through a reverse osmosis membrane, and recovering a permeate from the permeate side and recovering a concentrated aqueous sugar solution from the feed side;

wherein at least a part of the permeate from the reverse osmosis membrane is used as a hydrothermal treatment liquid, biomass-suspending liquid, enzyme-diluting liquid, acid-diluting liquid and/or alkali-diluting liquid in the Step (1).

The present invention can provide a method for producing a concentrated aqueous sugar solution, the method comprising hydrolyzing a cellulose-containing biomass to produce an aqueous sugar solution, treating the aqueous sugar solution with a microfiltration membrane and/or an ultrafiltration membrane to remove the biomass residue, and then concentrating the aqueous sugar solution by treatment with a reverse osmosis membrane to increase the sugar concentration, wherein the permeate discarded from the reverse osmosis membrane is recovered and reused to thereby save water.

EP 2 650 384 A1

## Description

### TECHNICAL FIELD

The present invention relates to a method for producing a concentrated aqueous sugar solution from a cellulose-containing biomass.

### BACKGROUND ART

The 20th century-the age of mass consumption and mass disposal-is now over, and construction of an environment-conscious society is demanded in the 21th century. As the problem of depletion of fossil resources and the problem of global warming have become more serious, promotion of utilization of biomass resources as recyclable resources has been more and more demanded.

At present, bioethanol, among the biomass resources, is extensively produced using sugar cane and maize as raw materials, in the United States, Brazil and the like. This is because sugar cane and maize contain plenty of sucrose and starch, and an aqueous sugar solution can hence be easily prepared from these, for use in fermentation. However, sugar cane and maize have been originally used as foods, and their use as the raw materials causes competition with foods and feeds, leading to sharp rises in the prices of the raw materials, which is seriously problematic. Thus, a process for efficiently producing an aqueous sugar solution from a non-food biomass such as a cellulose-containing biomass, and a process for efficiently using the obtained aqueous sugar solution as a fermentation feedstock for its conversion to an industrial material, need to be constructed in the future.

### PRIOR ART DOCUMENTS

[Patent Documents]

Examples of the method for producing an aqueous sugar solution from a cellulose-containing biomass include a method for producing an aqueous sugar solution using sulfuric acid. Methods using concentrated sulfuric acid for acid hydrolysis of cellulose and hemicellulose to produce an aqueous sugar solution have been disclosed (Patent Documents 1 and 2).

Further, as methods which do not use an acid, a method for producing an aqueous sugar solution by hydrolysis of a cellulose- containing biomass using subcritical water at about 250°C to 500°C (Patent Document 3), a method for producing an aqueous sugar solution by treating a cellulose- containing biomass with subcritical water followed by enzyme treatment (Patent Document 4), and a method for producing an aqueous sugar solution by hydrolyzing a cellulose-containing biomass with pressurized hot water at 240°C to 280°C followed by enzyme treatment (Patent Document 5) have been disclosed.

Examples of disclosed methods for removal of the biomass residue and concentration of the aqueous sugar solution include a method wherein a cellulose-containing biomass is hydrolyzed to produce an aqueous sugar solution and the produced solution is treated with a microfiltration membrane and/or ultrafiltration membrane to remove the biomass residue, followed by treating the resulting product with a nanofiltration membrane and/or reverse osmosis membrane to concentrate the aqueous sugar solution for increasing the sugar concentration (Patent Document 6).

[Patent Document 1] Japanese Translated PCT Patent Application Laid-open No. 11-506934
[Patent Document 2] JP 2005-229821 A
[Patent Document 3] JP 2003-212888 A
[Patent Document 4] JP 2001-95597 A
[Patent Document 5] JP 3041380 B
[Patent Document 6] WO2010/067785

[Non-patent Document]

A method for producing an aqueous sugar solution by subjecting a cellulose-containing biomass to hydrolysis treatment with dilute sulfuric acid and treating the resulting product with an enzyme such as cellulase has been disclosed (Non-patent Document 1).

[Non-patent Document 1] A. Aden et al., "Lignocellulosic Biomass to Ethanol Process Design and Economics Utilizing Co-Current Dilute Acid Prehydrolysis and Enzymatic Hydrolysis for Corn Stover" NREL Technical Report (2002)

## SUMMARY OF THE INVENTION

## PROBLEMS TO BE SOLVED BY THE INVENTION

However, the aqueous sugar solutions obtained by the techniques disclosed in Patent Documents 1 to 5 and Non- patent Document 1 contain a large amount of biomass residue, and the sugar concentration is low. Therefore, in order to use such an aqueous sugar solution as a fermentation feedstock by supplying it to a fermenter, it is necessary to remove the biomass residue by an appropriate solid- liquid separation treatment and then to increase the sugar concentration by concentrating the aqueous sugar solution.

In the case of the technique disclosed in Patent Document 6, the amount of water used in each step is still large because of, for example, washing of the biomass residue accumulated on a microfiltration membrane and/or ultrafiltration membrane. Therefore, the achievement of construction of an environment-conscious society requires construction of a water-saving process wherein the wastewater in each step is recovered and reused.

Accordingly, the present invention provides a method for producing a concentrated aqueous sugar solution, the method comprising hydrolyzing a cellulose- containing biomass to produce an aqueous sugar solution, treating the aqueous sugar solution with a microfiltration membrane and/or an ultrafiltration membrane to remove the biomass residue, and then concentrating the aqueous sugar solution by treatment with a reverse osmosis membrane to increase the sugar concentration, wherein water discarded is recovered and reused to thereby save water.

## MEANS FOR SOLVING THE PROBLEMS

In order to solve the above-described problem, the method of the present invention for producing a concentrated aqueous sugar solution comprises the following constitution.

That is, a method for producing a concentrated aqueous sugar solution using a cellulose-containing biomass as a raw material, the method comprising the steps of:

(1) hydrolyzing a cellulose-containing biomass to produce an aqueous sugar solution;
(2) filtering the aqueous sugar solution obtained in (1) through a microfiltration membrane and/or an ultrafiltration membrane, and recovering an aqueous sugar solution from the permeate side; and
(3) filtering the aqueous sugar solution obtained in (2) through a reverse osmosis membrane, and recovering a permeate from the permeate side and recovering a concentrated aqueous sugar solution from the feed side;
wherein at least a part of the permeate is used as at least one of a hydrothermal treatment liquid, biomass-suspending liquid, washing liquid, enzyme-diluting liquid, acid-diluting liquid and alkali-diluting liquid in the Step (1).

The method of the present invention for producing ethanol is a method for producing ethanol using a yeast from a concentrated aqueous sugar solution obtained by the above method.

In the method of the present invention for producing a concentrated aqueous sugar solution, in cases where the acetic acid concentration in the permeate from the reverse osmosis membrane is less than 1.5 g/L, the permeate is preferably used as at least one of a hydrothermal treatment liquid, enzyme-diluting liquid, acid-diluting liquid and alkali-diluting liquid in the Step (1), while in cases where the acetic acid concentration is not less than 1.5 g/L, the permeate is preferably used as a hydrothermal treatment liquid and/or washing liquid in the Step (1).

Further, in the method of the present invention for producing a concentrated aqueous sugar solution, the reverse osmosis membrane is preferably a composite membrane comprising polyamide as a functional layer.

In the method of the present invention for producing a concentrated aqueous sugar solution, the reverse osmosis membrane preferably has a salt rejection rate of not less than 90% when measurement is carried out using 500 mg/L saline at 0.76 MPa, 25°C and pH 6.5.

In the method of the present invention for producing a concentrated aqueous sugar solution, the microfiltration membrane and/or ultrafiltration membrane is/are preferably a hollow fiber membrane(s).

The method of the present invention for producing a concentrated aqueous sugar solution further preferably comprises the step of filtering the aqueous sugar solution obtained in the Step (2) through a nanofiltration membrane.

## EFFECT OF THE INVENTION

By the present invention wherein at least a part of water which has been discarded in the past is recovered and reused, it is possible to produce a concentrated aqueous sugar solution while suppressing the amount of water consumed. As a result, utilization of biomass resources as recyclable resources can be promoted, which in turn contributes to construction of an environment-conscious society.

BRIEF DESCRIPTION OF THE DRAWINGS

Fig. 1 is a schematic flow diagram showing an embodiment of the present invention.

BEST MODE FOR CARRYING OUT THE INVENTION

The present invention will now be described in more detail.

Examples of the cellulose-containing biomass used in the method of the present invention for producing a concentrated aqueous sugar solution include herbaceous biomasses such as bagasse, switchgrass, corn stover, rice straw and wheat straw; and woody biomasses such as trees and waste building materials. These cellulose-containing biomasses contain cellulose and hemicellulose, which are polysaccharides produced by dehydration condensation of sugars. By hydrolyzing such polysaccharides, aqueous sugar solutions which may be used as fermentation feedstocks can be produced.

The aqueous sugar solution of the present invention means an aqueous sugar solution obtained by hydrolysis of a cellulose- containing biomass. Sugars are generally classified, based on the degree of polymerization of monosaccharides, into monosaccharides such as glucose and xylose, oligosaccharides produced by dehydration condensation of 2 to 9 monosaccharides, and polysaccharides produced by dehydration condensation of not less than 10 monosaccharides. The aqueous sugar solution of the present invention means an aqueous sugar solution containing a monosaccharide (s) as a major component (s), and more particularly, the aqueous sugar solution of the present invention contains glucose and/or xylose as a major component (s) . Further, the aqueous sugar solution of the present invention also contains oligosaccharides such as cellobiose; and monosaccharides such as arabinose and mannose, although their amounts are small. Here, the term "containing a monosaccharide (s) as a major component (s) " means that a monosaccharide (s) constitute (s) not less than 80% by weight of the total weight of sugars such as monosaccharides, oligosaccharides and polysaccharides dissolved in water. Specific examples of the method for analyzing monosaccharides, oligosaccharides and polysaccharides dissolved in water include quantification by high- performance liquid chromatography (HPLC) based on comparison with a standard sample. Specific HPLC conditions are as follows: no use of a reaction liquid; use of Luna $NH_2$ (manufactured by Phenomenex, Inc.) as a column; mobile phase, ultrapure water: acetonitrile=25: 75; flow rate, 0.6 mL/min.; measurement time, 45 min.; detection method, RI (differential refractive index) ; temperature, 30°C.

Step (1) in the method of the present invention for producing a concentrated aqueous sugar solution, which is the step of hydrolyzing a cellulose-containing biomass to produce an aqueous sugar solution, will now be described.

When a cellulose-containing biomass is subjected to hydrolysis, the cellulose-containing biomass may be used as it is, or may be subjected to known treatment such as steaming, pulverization and blasting. By such treatment, the efficiency of hydrolysis can be enhanced.

The step of hydrolysis of the cellulose-containing biomass is not restricted, and specific examples the step mainly include 6 methods, that is, Procedure A: a method using only an acid; Procedure B: a method wherein acid treatment is carried out followed by use of an enzyme; Procedure C: a method using only hydrothermal treatment; Procedure D: a method wherein hydrothermal treatment is carried out followed by use of an enzyme; Procedure E: a method wherein alkaline treatment is carried out followed by use of an enzyme; and Procedure F: a method wherein ammonia treatment is carried out followed by use of an enzyme.

In Procedure A, an acid is used for the hydrolysis of a cellulose-containing biomass. Examples of the acid to be used include sulfuric acid, nitric acid and hydrochloric acid, and sulfuric acid is preferably used.

The concentration of the acid is not restricted, and an acid at a concentration of 0.1 to 99% by weight may be used. In cases where the concentration of the acid is 0.1 to 15% by weight, preferably 0.5 to 5% by weight, the reaction temperature is set within the range of 100 to 300°C, preferably 120 to 250°C, and the reaction time is set within the range of 1 second to 60 minutes. The number of times of treatment is not restricted, and 1 or more times of the above-described treatment may be carried out. In particular, in cases where the above-described treatment is carried out 2 or more times, the conditions for the first treatment may be different from those for the second and later treatments.

Further, in cases where the concentration of the acid is 15 to 95% by weight, preferably 60 to 90% by weight, the reaction temperature is set within the range of 10 to 100°C, and the reaction time is set within the range of 1 second to 60 minutes.

The number of times of the acid treatment is not restricted, and 1 or more times of the above-described treatment may be carried out. In particular, in cases where the above-described treatment is carried out 2 or more times, the conditions for the first treatment may be different from those for the second and later treatments.

Since the hydrolysate obtained by the acid treatment contains an acid such as sulfuric acid, neutralization is necessary for its use as a fermentation feedstock. The alkaline reagent to be used for the neutralization is not restricted, and preferably a monovalent alkaline reagent. This is because, in cases where both of the acid and alkaline components are salts having valencies of 2 or more, the salts precipitate in the liquid during the process of concentration of the liquid,

which may in turn cause fouling of the membrane.

In cases where a monovalent alkali is used, examples of the alkali include, but are not limited to, ammonia, sodium hydroxide and potassium hydroxide.

In cases where an alkaline reagent having a valency of 2 or more is used, it may be necessary to reduce the amounts of the acid and the alkali in order to avoid precipitation of a salt, or to employ a mechanism for removal of precipitates, or the like.

In general, in hydrolysis using an acid, hydrolysis first occurs in the hemicellulose component having low crystallinity, which is followed by degradation of the cellulose component having high crystallinity. Therefore, it is possible, by using an acid, to obtain a liquid containing a large amount of xylose derived from hemicellulose. In the acid treatment, by further subjecting the treated biomass solid content to a reaction at higher pressure and higher temperature than in the above treatment, the cellulose component having higher crystallinity can be further degraded to obtain a liquid containing a large amount of glucose derived from cellulose. By setting the two-stage step of hydrolysis, conditions for the hydrolysis which are suitable for hemicellulose and cellulose can be set, and hence the degradation efficiency and the sugar yield can be enhanced. Further, by keeping the aqueous sugar solution obtained under the first degradation conditions and the aqueous sugar solution obtained under the second degradation conditions isolated from each other, two types of aqueous sugar solutions containing monosaccharide components at different ratios in the hydrolysate can be produced. That is, it is also possible to separate an aqueous sugar solution under the first degradation conditions such that it contains xylose as the major component, and to separate an aqueous sugar solution under the second degradation conditions such that it contains glucose as the major component. By separating the monosaccharide components contained in the aqueous sugar solution as described above, fermentation using xylose in the aqueous sugar solution as a fermentation feedstock and fermentation using glucose in the aqueous sugar solution as a fermentation feedstock can be performed separately, so that microorganism species which are most suitable for the respective types of fermentation can be selected and employed. It should be noted that, by carrying out high-pressure high-temperature treatment with an acid for a long time, sugars derived from both of the hemicellulose component and the cellulose component may be obtained at once without separating these components.

In Procedure B, the treated liquid obtained in Procedure A is further subjected to enzymatic hydrolysis of the cellulose-containing biomass. The concentration of the acid to be used in Procedure B is preferably 0.1 to 15% by weight, more preferably 0.5 to 5% by weight. The reaction temperature may be set within the range of 100 to 300°C, preferably 120 to 250°C. The reaction time may be set within the range of 1 second to 60 minutes. The number of times of treatment is not restricted, and 1 or more times of the above-described treatment may be carried out. In particular, in cases where the above-described treatment is carried out 2 or more times, the conditions for the first treatment may be different from those for the second and later treatments.

Since the hydrolysate obtained by the acid treatment contains an acid such as sulfuric acid, neutralization is necessary for further performing hydrolysis reaction with an enzyme or for its use as a fermentation feedstock. The neutralization may be carried out in the same manner as the neutralization in Procedure A.

The enzyme is not restricted as long as it is an enzyme having cellulose-degrading activity, and commonly-used cellulases may be used. The enzyme is preferably a cellulase such as an exo-type cellulase or endo-type cellulase having an activity to degrade crystalline cellulose. Such a cellulase is preferably a cellulase produced by *Trichoderma. Trichoderma* is a genus of microorganisms classified as filamentous fungi, and they extracellularly secrete a large amount of various cellulases. The cellulase to be used in the present invention is preferably a cellulase derived from *Trichoderma reesei.* Further, as an enzyme to be used for the hydrolysis, β-glucosidase, which is a cellobiose-degrading enzyme, may be added in order to increase the production efficiency of glucose. The β-glucosidase may be used in combination with the above-mentioned cellulase for the hydrolysis. The β-glucosidase is not restricted, and is preferably derived from *Aspergillus.* The hydrolysis reaction using such an enzyme(s) is preferably carried out at a pH of about 3 to 7, more preferably at a pH of about 5. The reaction temperature is preferably 40 to 70°C.

In cases where the acid treatment is followed by enzymatic hydrolysis of the cellulose-containing biomass, it is preferred to carry out hydrolysis of hemicellulose having low crystallinity by the acid treatment in the first hydrolysis, followed by carrying out hydrolysis of cellulose having high crystallinity by using an enzyme in the second hydrolysis. By using the enzyme in the second hydrolysis, the step of hydrolysis of the cellulose-containing biomass can be allowed to proceed more efficiently. More particularly, in the first hydrolysis by an acid, hydrolysis of the hemicellulose component contained in the cellulose-containing biomass and partial degradation of lignin mainly occur, and the resulting hydrolysate is separated into an acid solution and the solid content containing cellulose. The solid content containing cellulose is then hydrolyzed by addition of an enzyme. Since the separated/recovered solution in dilute sulfuric acid contains, as a major component, xylose, which is a pentose, an aqueous sugar solution can be isolated by neutralization of the acid solution. Further, from the hydrolysis reaction product of the solid content containing cellulose, monosaccharide components containing glucose as a major component can be obtained. The aqueous sugar solution obtained by neutralization may also be mixed with the solid content, followed by adding an enzyme to the resulting mixture to carry out hydrolysis.

In Procedure C, an acid is not particularly added, and water is added such that the concentration of the cellulose-containing biomass becomes 0.1 to 50% by weight, followed by treatment at a temperature of 100 to 400°C for 1 second to 60 minutes. By carrying out the treatment under such a temperature condition, hydrolysis of cellulose and hemicellulose occurs. The number of times of treatment is not restricted, and the treatment may be carried out 1 or more times. In particular, in cases where the treatment is carried out 2 or more times, the conditions for the first treatment may be different from those for the second and later treatments.

In general, in hydrolysis employing hydrothermal treatment, hydrolysis first occurs in the hemicellulose component having low crystallinity, which is followed by degradation of the cellulose component having high crystallinity. Therefore, it is possible, by using hydrothermal treatment, to obtain a liquid containing a large amount of xylose derived from hemicellulose. Further, in the hydrothermal treatment, the cellulose component having higher crystallinity can be degraded by further subjecting the treated biomass solid content to a reaction at higher pressure and higher temperature than in the above treatment, to obtain a liquid containing a large amount of glucose derived from cellulose. By setting the two-stage step of hydrolysis, conditions for the hydrolysis which are suitable for hemicellulose and cellulose can be set, and the degradation efficiency and the sugar yield can be increased. Further, by keeping the aqueous sugar solution obtained under the first degradation conditions and the aqueous sugar solution obtained under the second degradation conditions isolated from each other, two types of aqueous sugar solutions containing monosaccharide components at different ratios in the hydrolysate can be produced. That is, it is also possible to separate an aqueous sugar solution under the first degradation conditions such that it contains xylose as the major component, and to separate an aqueous sugar solution under the second degradation conditions such that it contains glucose as the major component. By separating the monosaccharide components contained in the aqueous sugar solution as described above, fermentation using xylose in the aqueous sugar solution as a fermentation feedstock and fermentation using glucose in the aqueous sugar solution as a fermentation feedstock can be performed separately, so that microorganism species which are most suitable for the respective types of fermentation can be selected and employed.

In Procedure D, the treated liquid obtained in Procedure C is further subjected to enzymatic hydrolysis of the cellulose-containing biomass.

The enzyme to be used may be the same as the one used in Procedure B. The conditions for the enzyme treatment may also be the same as those in Procedure B.

In cases where hydrothermal treatment is followed by enzymatic hydrolysis of the cellulose-containing biomass, hemicellulose having low crystallinity is hydrolyzed by hydrothermal treatment in the first hydrolysis, and cellulose having high crystallinity is then hydrolyzed using an enzyme in the second hydrolysis. By using the enzyme in the second hydrolysis, the step of hydrolysis of the cellulose-containing biomass can be allowed to proceed more efficiently. More particularly, in the first hydrolysis by hydrothermal treatment, hydrolysis of the hemicellulose component contained in the cellulose-containing biomass and partial degradation of lignin mainly occur, and the resulting hydrolysate is separated into an aqueous solution and the solid content containing cellulose. The solid content containing cellulose is then hydrolyzed by addition of an enzyme. The separated/recovered solution contains xylose, which is a pentose, as a major component. Further, from the hydrolysis reaction product of the solid content containing cellulose, monosaccharide components containing glucose as a major component can be obtained. Further, the aqueous solution obtained by the hydrothermal treatment may also be mixed with the solid content, followed by adding an enzyme to the resulting mixture to carry out hydrolysis.

In Procedure E, the alkali to be used is more preferably sodium hydroxide or calcium hydroxide. These alkalis may be added to the cellulose-containing biomass such that their concentrations are within the range of 0.1 to 60% by weight, and the treatment may be carried out at a temperature within the range of 100 to 200°C, preferably 110 to 180°C. The number of times of treatment is not restricted, and 1 or more times of the above-described treatment may be carried out. In particular, in cases where the above-described treatment is carried out 2 or more times, the conditions for the first treatment may be different from those for the second and later treatments.

Since the treated product obtained by the alkaline treatment contains an alkali such as sodium hydroxide, it needs to be neutralized in order to be further subjected to hydrolysis reaction using an enzyme. The acid reagent to be used for the neutralization is not restricted, and is preferably a monovalent acid reagent. This is because, in cases where both of the acid and alkaline components are salts having valencies of 2 or more, the salts precipitate in the liquid during the process of concentration of the liquid, which may in turn cause fouling of the membrane.

In cases where a monovalent acid is used, examples of the acid include, but are not limited to, nitric acid and hydrochloric acid.

In cases where an acid reagent having a valency of 2 or more is used, it may be necessary, for example, to reduce the amounts of the acid and the alkali in order to avoid precipitation of a salt, or to employ a mechanism for removal of precipitates. In cases where an acid having a valency of 2 or more is used, the acid is preferably sulfuric acid or phosphoric acid.

The enzyme to be used may be the same as the one used in Procedure B. The conditions for the enzyme treatment may also be the same as those in Procedure B.

In cases where the alkaline treatment is followed by enzymatic hydrolysis of the cellulose-containing biomass,

the cellulose-containing biomass is mixed with an aqueous solution containing an alkali and the resulting mixture is heated to remove the lignin component around the hemicellulose and cellulose components for making the hemicellulose and cellulose components reactive, followed by carrying out enzymatic hydrolysis of hemicellulose having low crystallinity and cellulose having high crystallinity which remained undegraded during the alkaline treatment. More particularly, in the alkaline treatment, hydrolysis of a part of the hemicellulose component contained in the cellulose-containing biomass and partial degradation of lignin mainly occur, and the resulting hydrolysate is separated into an alkaline solution and the solid content containing cellulose. The solid content containing cellulose is then hydrolyzed by adjusting the pH and adding an enzyme thereto. In cases where the concentration of the alkaline solution is low, the hydrolysis may be carried out by just adding the enzyme after neutralization, without separation of the solid content. From the hydrolysis reaction product of the solid content containing cellulose, monosaccharide components containing glucose and xylose as major components can be obtained. Further, since the separated/recovered alkaline solution contains, as a major component, xylose, which is a pentose, in addition to lignin, an aqueous sugar solution can also be isolated by neutralization of the alkaline solution. Further, the aqueous sugar solution obtained by neutralization may be mixed with the solid content, followed by adding an enzyme to the resulting mixture to carry out hydrolysis.

The conditions for the ammonia treatment in Procedure F are based on the treatment conditions described in JP 2008-161125 A and JP 2008-535664 A. For example, ammonia is added to the cellulose-containing biomass at a concentration within the range of 0.1 to 15% by weight with respect to the cellulose-containing biomass, and the treatment is carried out at 4°C to 200°C, preferably 90°C to 150°C. The ammonia to be added may be in the state of either liquid or gas. Further, the form of the ammonia to be added may be either pure ammonia or aqueous ammonia. The number of times of treatment is not restricted, and 1 or more times of the treatment may be carried out. In particular, in cases where the treatment is carried out 2 or more times, the conditions for the first treatment may be different from those for the second and later treatments.

The treated product obtained by the ammonia treatment needs to be subjected to neutralization of ammonia or removal of ammonia in order to further carry out hydrolysis reaction using an enzyme. The acid reagent to be used for the neutralization is not restricted. Examples of the acid reagent include hydrochloric acid, nitric acid and sulfuric acid, and the acid reagent is preferably sulfuric acid in view of avoiding corrosion of process piping and avoiding inhibition of fermentation. The ammonia can be removed by maintaining the ammonia-treated product under reduced pressure to evaporate the ammonia into the gas state. The removed ammonia may be recovered and reused.

It is known that, in hydrolysis using an enzyme after ammonia treatment, the crystal structure of cellulose is changed by the ammonia treatment and the resulting crystal structure allows the enzyme reaction to occur easily. Therefore, by allowing the enzyme to act on the solid content after such ammonia treatment, hydrolysis can be carried out efficiently. The enzyme to be used may be the same as the one used in Procedure B. The conditions for the enzyme treatment may also be the same as those in Procedure B.

In cases where aqueous ammonia is used, the water component, in addition to ammonia, may give an effect similar to Procedure C (hydrothermal treatment), and hydrolysis of hemicellulose and degradation of lignin may occur. In cases where the treatment with aqueous ammonia is followed by enzymatic hydrolysis of the cellulose-containing biomass, the cellulose-containing biomass is mixed with an aqueous solution containing ammonia and the resulting mixture is heated to remove the lignin component around the hemicellulose and cellulose components for making the hemicellulose and cellulose components reactive, followed by carrying out enzymatic hydrolysis of hemicellulose having low crystallinity and cellulose having high crystallinity which remained undegraded during the hydrothermal process in the ammonia treatment. More particularly, in the treatment with aqueous ammonia, hydrolysis of a part of the hemicellulose component contained in the cellulose-containing biomass and partial decomposition of lignin mainly occur, and the resulting hydrolysate is separated into aqueous ammonia and the solid content containing cellulose. The solid content containing cellulose is then hydrolyzed by adjusting the pH and adding an enzyme thereto. In cases where the concentration of ammonia is as high as about 100%, a large portion of the ammonia may be removed by degassing, followed by neutralization of the resultant and addition of an enzyme thereto without separation of the solid content, to carry out hydrolysis. From the hydrolysis reaction product of the solid content containing cellulose, monosaccharide components containing glucose and xylose as major components can be obtained. Further, since the separated/recovered aqueous ammonia contains, as a major component, xylose, which is a pentose, in addition to lignin, an aqueous sugar solution can also be isolated by neutralizing the alkaline solution. Further, the aqueous sugar solution obtained by neutralization may be mixed with the solid content, followed by adding an enzyme to the resulting mixture, to carry out hydrolysis.

The aqueous sugar solution obtained in the Step (1) contains not only sugars, but also the biomass residue containing colloidal components, suspended matters, fine particles and the like. Examples of such components constituting the biomass residue include, but are not limited to, lignin, tannin, silica, calcium and undegraded cellulose.

Step (2) of the method of the present invention for producing a concentrated aqueous sugar solution, wherein the aqueous sugar solution obtained in Step (1) is filtered through a microfiltration membrane and/or an ultrafiltration membrane and recovered from the permeate side, is described below.

The microfiltration membrane used in the present invention is a membrane having an average pore size of 0.01

μm to 5 mm, which is called microfiltration, MF membrane or the like for short. The ultrafiltration membrane used in the present invention is a membrane having a molecular weight cutoff of 1,000 to 200,000, which is called UF membrane or the like for short. Here, in the ultrafiltration membrane, the pore size is too small to measure the size of each pore on the membrane surface under the electron microscope or the like, so that the molecular weight cutoff is used as an index of the size of the pore instead of the average pore size. As is described in p. 92 of Membrane Experiment Series, Vol. III, Artificial Membrane, The Membrane Society of Japan ed., editorial committee members: Shoji Kimura, Shin-ichi Nakao, Haruhiko Ohya and Tsutomu Nakagawa (1993, Kyoritsu Shuppan Co., Ltd.) that "The curve obtained by plotting the molecular weight of the solute along the abscissa and the blocking rate along the ordinate is called the molecular weight cutoff curve. The molecular weight with which the blocking rate reaches 90% is called the molecular weight cutoff.", the molecular weight cutoff is known as an index representing the membrane performance of an ultrafiltration membrane.

The material of the microfiltration membrane or ultrafiltration membrane is not restricted as long as removal of the biomass residue described above is possible therewith, and examples of the material include organic materials such as cellulose, cellulose ester, polysulfone, polyether sulfone, chlorinated polyethylene, polypropylene, polyolefin, polyvinyl alcohol, polymethyl methacrylate, polyvinylidene fluoride and polytetrafluoroethylene; metals such as stainless steel; and inorganic materials such as ceramics. The material of the microfiltration membrane or ultrafiltration membrane may be appropriately selected depending on the properties of the hydrolysate and/or the running cost, and the material is preferably an organic material in view of ease of handling, more preferably chlorinated polyethylene, polypropylene, polyvinylidene fluoride, polysulfone or polyether sulfone.

Further, by filtering the aqueous sugar solution obtained in the Step (1) especially through an ultrafiltration membrane, the enzyme which was used for saccharification can be recovered from the feed side. The recovery process of the enzyme will now be described. The enzyme used in the hydrolysis has a molecular weight within the range of 10, 000 to 100, 000, and, by using an ultrafiltration membrane having a molecular weight cutoff with which permeation of the enzyme can be blocked, the enzyme can be recovered from the fraction in the feed side. Preferably, by using an ultrafiltration membrane having a molecular weight cutoff within the range of 10, 000 to 30, 000, the enzyme to be used for hydrolysis can be efficiently recovered. The form of the ultrafiltration membrane used is not restricted, and may be in the form of either a flat membrane or a hollow fiber membrane. By reusing the recovered enzyme in the hydrolysis in Step (1), the amount of enzyme to be used may be reduced. When such filtration of an aqueous sugar solution through an ultrafiltration membrane is carried out, the aqueous sugar solution is preferably preliminarily processed by being passed through a microfiltration membrane to remove water- soluble polymers and colloidal components in the biomass residue, which easily cause membrane fouling in an ultrafiltration membrane.

The operation of filtration may be multistage filtration wherein a microfiltration membrane(s) and/or ultrafiltration membrane(s) is/are used two or more times for efficient removal of water-soluble polymers and colloidal components, and the material and the properties of each membrane used for the filtration are not restricted.

For example, in a method wherein filtration through a microfiltration membrane is performed and then the obtained filtrate is further filtered through an ultrafiltration membrane, it is possible to remove colloidal components having sizes of not more than several ten nanometers, which cannot be removed with a microfiltration membrane; water-soluble macromolecular components derived from lignin (tannin); sugars which were hydrolyzed into oligosaccharides and polysaccharides but are still in the middle of the process of degradation into monosaccharides; and the enzyme used for hydrolysis of sugars.

Although the microfiltration membrane or ultrafiltration membrane in the present invention may be in the form of either a hollow fiber membrane or a flat membrane, a hollow fiber membrane is preferably used in cases where the later-mentioned backwashing is carried out.

Step (3) of the method of the present invention for producing a concentrated aqueous sugar solution, wherein the aqueous sugar solution obtained in Step (2) is filtered through a reverse osmosis membrane, and a permeate is recovered from the permeate side and a concentrated aqueous sugar solution is recovered from the feed side, is described below.

The term "filtered through a reverse osmosis membrane" in the present invention means that the aqueous sugar solution obtained by hydrolysis of a cellulose-containing biomass is filtered through a microfiltration membrane and/or ultrafiltration membrane and the aqueous sugar solution recovered from the permeate side is filtered through a reverse osmosis membrane to block or separate an aqueous sugar solution of dissolved sugars, especially monosaccharides such as glucose and xylose, into the feed side.

In terms of the removal performance of the reverse osmosis membrane used in the present invention, the membrane has a salt rejection rate of preferably not less than 90%, more preferably not less than 95%, still more preferably not less than 99% when measurement is carried out using 500 mg/L saline at 0.76 MPa, 25°C and pH 6.5. The higher the salt rejection rate of the reverse osmosis membrane, the more efficiently sugars can be concentrated in the aqueous sugar solution. The rejection rate of a reverse osmosis membrane can be calculated using the concentrations of the subject compound (salt, monosaccharide or the like) contained in the feed side and the permeate side, according

to the Equation (I) below.

$$\text{Rejection rate (\%)} = (1 - \text{concentration of subject compound in permeate side} / \text{concentration of subject compound in feed side}) \times 100 \ldots \text{(I)}$$

The analysis method for measurement of the concentrations of the subject compound in Equation (I) is not restricted as long as the method enables highly accurate and reproducible measurement, and the method is preferably use of ion chromatography, high-frequency inductively coupled plasma emission spectrometry (ICP), conductivity meter or the like in cases of a salt; or use of high-performance liquid chromatography, refractometer or the like in cases of a monosaccharide.

In terms of the permeability of the reverse osmosis membrane used in the present invention, the membrane shows a permeation flow rate per unit membrane area of preferably not less than 0.3 $m^3/m^2$/day, more preferably not less than 0.6 $m^3/m^2$/day, still more preferably not less than 0.9 $m^3/m^2$/day when measurement is carried out using 500 mg/L saline at 0.76 MPa, 25°C and pH 6.5. The higher the permeation flow rate per unit membrane area of the reverse osmosis membrane, the more efficiently sugars can be concentrated from the aqueous sugar solution. The permeation flow rate per unit membrane area (membrane permeation flux or flux) of a reverse osmosis membrane can be determined by measuring the amount of liquid permeated, sampling time of the permeated liquid and the membrane area, and performing calculation according to the Equation (II) below.

$$\text{Membrane permeation flux } (m^3/m^2/\text{day}) = \text{amount of liquid permeated} / \text{membrane area} / \text{liquid sampling time} \ldots \text{(II)}$$

In terms of the material of the reverse osmosis membrane used in the present invention, examples of the membrane include a composite membrane comprising a cellulose acetate polymer as a functional layer (which may be hereinafter referred to as cellulose acetate reverse osmosis membrane) and a composite membrane comprising a polyamide as a functional layer (which may be hereinafter referred to as polyamide reverse osmosis membrane). Examples of the cellulose acetate polymer herein include polymers prepared with organic acid esters of cellulose such as cellulose acetate, cellulose diacetate, cellulose triacetate, cellulose propionate and cellulose butyrate, which may be used individually, as a mixture, or as a mixed ester. Examples of the polyamide include linear polymers and cross-linked polymers constituted by aliphatic and/or aromatic diamine monomers.

Among these, a polyamide reverse osmosis membrane is preferred since it has excellent potential with high pressure resistance, high permeability and high solute removal performance. For maintenance of durability against the operation pressure, high permeability and high blocking performance, the membrane preferably has a polyamide functional layer which is retained by a support made of a porous membrane and/or a non-woven fabric. The polyamide reverse osmosis membrane is preferably a composite semipermeable membrane having a functional layer on a support, which functional layer is composed of a cross-linked polyamide obtained by polycondensation of a polyfunctional amine and a polyfunctional acid halide.

In the polyamide reverse osmosis membrane, preferred examples of the carboxylic component of the monomers constituting the polyamide include aromatic carboxylic acids such as trimesic acid, benzophenone tetracarboxylic acid, trimellitic acid, pyromellitic acid, isophthalic acid, terephthalic acid, naphthalenedicarboxylic acid, diphenylcarboxylic acid and pyridinecarboxylic acid, and, in view of solubility to the film-forming solvent, trimesic acid, isophthalic acid or terephthalic acid, or a mixture thereof is more preferred.

Preferred examples of the amine component of the monomers constituting the polyamide include: primary diamines having an aromatic ring (s), such as m- phenylenediamine, p- phenylenediamine, benzidine, methylenebisdianiline, 4, 4'- diaminobiphenyl ether, dianisidine, 3, 3', 4- triaminobiphenyl ether, 3, 3', 4, 4'- tetraaminobiphenyl ether, 3, 3'- dioxybenzidine, 1, 8- naphthalenediamine, m (p)- monomethylphenylenediamine, 3, 3'- monomethylamino- 4, 4'- diaminobiphenyl ether, 4, N, N'- (4- aminobenzoyl)- p (m)- phenylenediamine- 2, 2'- bis (4- aminophenyl benzimidazole), 2, 2'- bis (4- aminophenyl benzoxazole), 2, 2'- bis (4- aminophenyl benzothiazole) ; and secondary diamines such as piperazine and piperidine and derivatives thereof. In particular, a reverse osmosis membrane having a functional layer composed of a cross- linked polyamide containing m- phenylenediamine and/or p- phenylenediamine as monomers is preferably used because of its high pressure resistance and durability as well as heat resistance and chemical resistance.

Specific examples of the reverse osmosis membrane used in the present invention include: polyamide reverse

osmosis membrane modules manufactured by TORAY INDUSTRIES, INC., SU-710, SU-720, SU-720F, SU-710L, SU-720L, SU-720LF, SU-720R, SU-710P, SU-720P, TMG10, TMG20-370 and TMG20-400, which are low-pressure type modules, as well as SU-810, SU-820, SU-820L and SU-820FA, which are high-pressure type modules; cellulose acetate reverse osmosis membranes manufactured by the same manufacturer, SC-L100R, SC-L200R, SC-1100, SC-1200, SC-2100, SC-2200, SC-3100, SC-3200, SC-8100 and SC-8200; NTR-759HR, NTR-729HF, NTR-70SWC, ES10-D, ES20-D, ES20-U, ES15-D, ES15-U and LF10-D, manufactured by Nitto Denko Corporation; RO98pHt, RO99, HR98PP and CE4040C-30D, manufactured by Alfa-Laval; GE Sepa, manufactured by GE; and BW30-4040, TW30-4040, XLE-4040, LP-4040, LE-4040, SW30-4040 and SW30HRLE-4040, manufactured by FilmTec Corporation.

In the filtration through a reverse osmosis membrane, the aqueous sugar solution obtained in Step (2) is preferably supplied to the reverse osmosis membrane at a pressure within the range of 1 MPa to 8 MPa. In cases where the pressure is within the above-described preferred range, the membrane permeation rate does not decrease, while there is no risk of damaging of the membrane. Further, in cases where the filtration pressure is 2 MPa to 7 MPa, the membrane permeation flux is high, so that the sugar solution can be allowed to permeate efficiently, and there is hardly the risk of damaging of the membrane, which is more preferred. The filtration pressure is especially preferably 3 MPa to 6 MPa.

The sugar components contained in the concentrated aqueous sugar solution obtained from the feed side of the reverse osmosis membrane are sugars derived from the cellulose-containing biomass, and, essentially, they are not largely different from the sugar components obtained by the hydrolysis in Step (1). That is, the monosaccharides contained in the concentrated aqueous sugar solution of the present invention are constituted by glucose and/or xylose as a major component(s). The ratio between glucose and xylose varies depending on the step of hydrolysis in Step (1). That is, in cases where hydrolysis was performed for mainly hemicellulose, xylose is the major monosaccharide component, while in cases where only the cellulose component was separated after degradation of hemicellulose and subjected to hydrolysis, glucose is the major monosaccharide component. Further, in cases where the cellulose component was not especially separated after degradation of hemicellulose, glucose and xylose are contained as major monosaccharide components.

Before passing the aqueous sugar solution through a reverse osmosis membrane, the solution may be concentrated using a concentrating apparatus such as an evaporator, or may be further concentrated by filtration through a separation membrane. In view of reducing the energy for concentration, the step of filtering the solution through a separation membrane to further concentrate the concentrated aqueous sugar solution may be preferably employed. The membrane used in this concentration step is a membrane filter that removes ions and low-molecular-weight molecules using as the driving force a pressure difference larger than the osmotic pressure of the liquid to be treated, and examples of the membrane which may be used include cellulose membranes such as those made of cellulose acetate and membranes produced by polycondensing a polyfunctional amine compound and a polyfunctional acid halide to provide a separation functional layer made of a polyamide on a microporous support membrane. In order to suppress dirt, that is, fouling, on the surface of the separation membrane, it is also preferred to employ, for example, a low-fouling membrane to be used for mainly sewage treatment, which is prepared by covering the surface of a separation functional layer made of a polyamide with an aqueous solution of a compound having at least one reactive group reactive with an acid halide group to form covalent bonds between acid halide groups remaining on the surface of the separation functional layer and the reactive group(s). Specific examples of the separation membrane to be used for the concentration are the same as those for the above-described reverse osmosis membrane and the later-described nanofiltration membrane.

In Step (3), the aqueous sugar solution obtained in Step (2) is filtered through a reverse osmosis membrane and a concentrated aqueous sugar solution is recovered from the feed side. The present invention is characterized in that at least a part of the permeate obtained from the permeate side of the reverse osmosis membrane is further used in the Step (1). That is, the present invention is characterized in that the permeate from the reverse osmosis membrane is not discarded as it is, and at least a part thereof is recovered and reused.

The quality of the permeate from the reverse osmosis membrane used in the present invention is dependent on the quality of the aqueous sugar solution supplied to the reverse osmosis membrane, the removal performance of the reverse osmosis membrane and the filtration conditions for the reverse osmosis membrane. However, compared to the aqueous sugar solution obtained by the Steps (1) and (2) described above, the concentrations of the biomass residue and sugars are low, and the permeate is sufficiently clear. Therefore, at least a part of the permeate from the reverse osmosis membrane may be used as the processing water for the Step (1). The processing water herein means water which is used by being directly mixed with the raw material, and specific examples of the processing water include a hydrothermal treatment liquid, biomass-diluting liquid, washing liquid, acid-diluting liquid and alkali/ammonia-diluting liquid.

In Procedure A, which is a method using only an acid, the permeate may be used as an acid-diluting liquid; in Procedure B, which is a method wherein acid treatment is carried out followed by use of an enzyme, the permeate may be used as an acid-diluting liquid and an aqueous enzyme solution; in Procedure C, which is a method using only hydrothermal treatment, the permeate may be used as a hydrothermal treatment liquid; in Procedure D, which is a

method wherein hydrothermal treatment is carried out followed by use of an enzyme, the permeate may be used as a hydrothermal treatment liquid and an aqueous enzyme solution; in Procedure E, which is a method wherein alkaline treatment is carried out followed by use of an enzyme, the permeate may be used as an alkali-diluting liquid and an aqueous enzyme solution; and in Procedure F, which is a method wherein ammonia treatment is carried out followed by use of an enzyme, the permeate may be used as an ammonia-diluting liquid and an aqueous enzyme solution. Depending on the procedure, the permeate may also be used as a biomass-suspending liquid for preliminarily suspending the biomass in water in order to increase the efficiency of hydrolysis reaction of the cellulose-containing biomass.

Thus, the use of the permeate from the reverse osmosis membrane may be determined in consideration of the amount of permeate from the reverse osmosis membrane, and the energy efficiency and the cost of the whole system. The use of the permeate from the reverse osmosis membrane may be preliminarily determined, or may be changed depending on changes in the raw material and the production conditions.

The use of the permeate from the reverse osmosis membrane is preferably determined based on the acetic acid concentration in the permeate. Preferably, in the Step (1), in cases where the acetic acid concentration in the permeate is less than 1.5 g/L, the permeate is used as at least one of the enzyme-diluting liquid, acid-diluting liquid and alkali-diluting liquid, while in cases where the acetic acid concentration is not less than 1.5 g/L, the permeate is used as a hydrothermal treatment liquid, and solid-liquid separation is carried out after the hydrothermal treatment.

The acetic acid concentration in the permeate can be measured by a known method. Examples of the measurement method include, but are not limited to, HPLC using an anion-exchange column.

At least a part of the permeate from the reverse osmosis membrane may be used as the washing liquid in the Step (1) and/or Step (2). The washing liquid herein means water which is used without being directly mixed with the raw material, and specific examples of the washing liquid include liquids to be used for rinsing or washing of the solid-liquid separation device or for rinsing or washing of the microfiltration membrane and/or ultrafiltration membrane.

Further, since washing of the biomass residue deposited on the microfiltration membrane and/or ultrafiltration membrane requires a large amount of water, it is preferred to use at least a part of the permeate from the reverse osmosis membrane as a washing liquid for the microfiltration membrane and/or ultrafiltration membrane. In terms of the method for washing the microfiltration membrane and/or ultrafiltration membrane, water is circulated from the primary side of the microfiltration membrane and/or ultrafiltration membrane, or water is circulated from the secondary side in the reverse direction. The latter method is the so called backwashing. In the present invention, for efficient removal of the biomass residue accumulated on the microfiltration membrane and/or ultrafiltration membrane, at least a part of the permeate from the reverse osmosis membrane is preferably used as a backwashing liquid for the microfiltration membrane and/or ultrafiltration membrane.

The amount of use and the utilization rate of the permeate from the reverse osmosis membrane in the Step (1) may be determined in consideration of the energy efficiency and the cost of the whole system. The amount of use and the utilization rate of the permeate from the reverse osmosis membrane may be preliminarily determined, or may be changed depending on changes in the raw material and production conditions. In order to allow production of a water-saving effect by recovery/reuse of the permeate from the reverse osmosis membrane, preferably 20 to 100% by weight, more preferably 40 to 100% by weight, still more preferably 60 to 100% by weight of the obtained permeate is utilized.

The step of filtering the aqueous sugar solution obtained in Step (2) through a nanofiltration membrane, and recovering a permeate from the permeate side and recovering a refined sugar solution from the feed side is described below.

In the present invention, "filtering through a nanofiltration membrane" means that an aqueous sugar solution obtained by hydrolyzing a cellulose-containing biomass is filtered through a microfiltration membrane and/or ultrafiltration membrane, and the aqueous sugar solution recovered from the permeate side is filtered through a nanofiltration membrane to block or separate an aqueous sugar solution of dissolved sugars, especially monosaccharides such as glucose and xylose, into the feed side, while removing or reducing fermentation-inhibiting substances by allowing them to permeate into the permeate side.

The "fermentation-inhibiting substances" herein means compounds which are produced by hydrolysis of a cellulose-containing biomass and have inhibitory actions as mentioned above during the step of fermentation using a refined sugar solution obtained by the production method of the present invention. The fermentation-inhibiting substances are produced especially during the step of acid treatment of the cellulose-containing biomass, and roughly classified into organic acids, furan compounds and phenolic compounds.

Examples of the organic acids include acetic acid, formic acid and levulinic acid. Examples of the furan compounds include furfural and hydroxymethylfurfural (HMF). Such organic acids and furan compounds are products produced by degradation of glucose and xylose, which are monosaccharides.

Specific examples of the phenolic compounds include vanillin, acetovanillin, vanillic acid, syringic acid, gallic acid, coniferyl aldehyde, dihydroconiferyl alcohol, hydroquinone, catechol, acetoguaicone, homovanillic acid, 4- hydroxybenzoic acid, and 4- hydroxy- 3- methoxyphenyl derivatives (Hibbert’s ketones) . These compounds are derived from lignin and lignin precursors.

Further, in cases where a waste building material, plywood or the like is used as the cellulose- containing biomass, components such as adhesives and paints used in the lumbering process may be contained as fermentation-inhibiting substances. Examples of the adhesives include urea resins, melamine resins, phenol resins, and urea/ melamine copolymers. Examples of fermentation- inhibiting substances derived from such adhesives include acetic acid, formic acid and formaldehyde.

In evaluation of the removal performance of the nanofiltration membrane used in the present invention in terms of the salt removal performance, saline is used for evaluation of the monovalent ion-removal performance, and an aqueous magnesium sulfate solution is used for evaluation of the divalent ion removal performance. When 500 mg/L saline is used and measurement is carried out at 0.34 MPa, 25°C and pH 6.5, the membrane has a salt rejection rate of preferably 10% to 80%, more preferably 10% to 70%, still more preferably 10% to 60%. The higher the salt rejection rate of the nanofiltration membrane in terms of saline, the more easily sugars can be concentrated from the aqueous sugar solution. However, in cases where the salt rejection rate is too high, efficient removal of fermentation-inhibiting substances is difficult. When 500 mg/L aqueous magnesium sulfate solution is used and measurement is carried out at 0.34 MPa, 25°C and pH 6.5, the membrane has a salt rejection rate of preferably 80% to 100%, more preferably 85% to 100%, still more preferably 90% to 100%. The higher the salt rejection rate of the nanofiltration membrane in terms of the aqueous magnesium sulfate solution, the more efficiently sugars can be purified from the aqueous sugar solution. In particular, for efficient purification of sugars from the aqueous sugar solution, the nanofiltration membrane preferably blocks sugars in the feed side and allows permeation of fermentation-inhibiting substances to the permeate side. In view of this, the nanofiltration membrane preferably has a low salt rejection rate in terms of monovalent ions and high salt rejection rate in terms of divalent ions. The nanofiltration membrane especially preferably has a salt rejection rate of 10% to 60% based on the estimation using saline, and a salt rejection rate of 90% to 100% based on the estimation using an aqueous magnesium sulfate solution. The rejection rate of a nanofiltration membrane can be calculated according to the Equation (III) below, based on the concentrations of the subject compound (salt, monosaccharide or the like) contained in the feed side and the permeate side.

$$\text{Rejection rate (\%)} = (1 - \text{concentration of subject compound in permeate side} / \text{concentration of subject compound in feed side}) \times 100 \ \ldots \ \text{(III)}$$

The analysis method for measurement of the concentrations of the subject compound in Equation (III) is not restricted as long as the method enables highly accurate and reproducible measurement, and the method is preferably use of ion chromatography, high-frequency inductively coupled plasma emission spectrometry (ICP), conductivity meter or the like in cases of a salt; or use of high-performance liquid chromatography, refractometer or the like in cases of a monosaccharide.

In terms of the permeability of the nanofiltration membrane used in the present invention, the membrane shows a permeation flow rate per unit membrane area of preferably not less than 0.5 $m^3/m^2$/day, more preferably not less than 0.6 $m^3/m^2$/day, still more preferably not less than 0.7 $m^3/m^2$/day when measurement is carried out using 500 mg/L saline at 0.34 MPa, 25°C and pH 6.5. The higher the permeation flow rate per unit membrane area of the nanofiltration membrane, the more efficiently sugars can be purified from the aqueous sugar solution. The permeation flow rate per unit membrane area (membrane permeation flux or flux) of a nanofiltration membrane can be determined by measuring the amount of liquid permeated, sampling time of the permeated liquid and the membrane area, and performing calculation according to the Equation (IV) below.

$$\text{Membrane permeation flux } (m^3/m^2/\text{day}) = \text{amount of liquid permeated} / \text{membrane area} / \text{liquid sampling time} \ \ldots \ \text{(IV)}$$

Examples of the material of the nanofiltration membrane which may be used in the present invention include macromolecular materials such as cellulose acetate polymers, polyamides, polyesters, polyimides and vinyl polymers. The membrane is not restricted to a membrane constituted by only one of the materials, and may be a membrane comprising a plurality of materials. In terms of the structure of the membrane, the membrane may be either an asymmetric membrane which has a dense layer on at least one side and micropores having pore sizes that gradually increase in the direction from the dense layer toward the inside of the membrane or the other side of the membrane, or a composite membrane which has a very thin functional layer formed by another material on the dense layer of an asymmetric membrane.

Examples of the composite membrane which may be used include the composite membrane described in JP 62-201606 A, which has a nanofilter composed of a polyamide functional layer on a support membrane comprising polysulfone as a membrane material.

Among these, a composite membrane having a functional layer composed of a polyamide is preferred since it has a high pressure resistance, high permeability and high solute-removal performance, which make the membrane highly potential. For maintenance of durability against the operation pressure, and high permeability and blocking performance, a membrane having a structure in which a polyamide is used as a functional layer, which layer is retained by a support comprising a porous membrane and/or a non-woven fabric, is suitable. Further, as a polyamide semipermeable membrane, a composite semipermeable membrane having, on a support, a functional layer of a cross-linked polyamide obtained by polycondensation reaction between a polyfunctional amine and a polyfunctional acid halide is suitable.

In the nanofiltration membrane having a functional layer composed of a polyamide, preferred examples of the carboxylic acid component of the monomers constituting the polyamide include aromatic carboxylic acids such as trimesic acid, benzophenone tetracarboxylic acid, trimellitic acid, pyromellitic acid, isophthalic acid, terephthalic acid, naphthalene dicarboxylic acid, diphenylcarboxylic acid and pyridinecarboxylic acid. In view of solubility to film-forming solvents, trimesic acid, isophthalic acid and terephthalic acid, and mixtures thereof are more preferred.

Preferred examples of the amine component of the monomers constituting the polyamide include primary diamines having an aromatic ring (s), such as m- phenylenediamine, p- phenylenediamine, benzidine, methylene bis dianiline, 4, 4'- diaminobiphenylether, dianisidine, 3, 3', 4- triaminobiphenylether, 3, 3', 4, 4'- tetraaminobiphenylether, 3, 3'- dioxybenzidine, 1, 8- naphthalenediamine, m (p)- monomethylphenylenediamine, 3, 3'- monomethylamino- 4, 4'- diaminobiphenylether, 4, N, N'- (4- aminobenzoyl)- p (m)- phenylenediamine- 2, 2'- bis (4- aminophenylbenzoimidazole), 2, 2'- bis (4- aminophenylbenzooxazole) and 2, 2'- bis (4- aminophenylbenzothiazole) ; and secondary diamines such as piperazine, piperidine and derivatives thereof. Among these, a nanofiltration membrane having a functional layer composed of a cross- linked polyamide comprising piperazine or piperidine as monomers is preferably used since it has heat resistance and chemical resistance in addition to pressure resistance and durability. The polyamide more preferably contains as a major component the cross- linked piperazine polyamide or cross- linked piperidine polyamide and further contains a constituting component represented by the Chemical Formula (1) below.

[Chemical Formula 1]

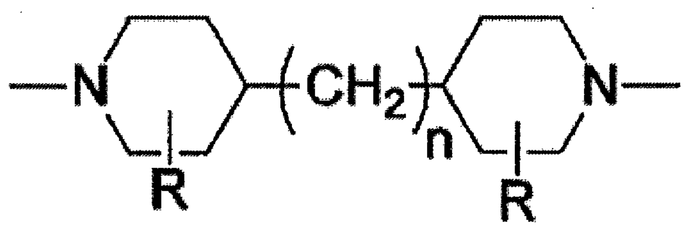

The polyamide still more preferably contains a cross-linked piperazine polyamide as a major component and further contains a constituting component represented by the Chemical Formula (1).

Further, preferably, in the Chemical Formula (1), n=3. Examples of the nanofiltration membrane having a functional layer composed of a polyamide containing a cross- linked piperazine polyamide as a major component and further containing a constituting component represented by the Chemical Formula (1) include the one described in JP 62- 201606 A, and specific examples of the membrane include UTC60 manufactured by TORAY INDUSTRIES, INC., which is a cross- linked piperazine polyamide nanofiltration membrane having a functional layer composed of a polyamide containing a cross- linked piperazine polyamide as a major component and further containing a constituting component represented by the Chemical Formula (1) wherein n=3.

A nanofiltration membrane is generally used as a spiral-wound membrane element, and the nanofiltration membrane used in the present invention is also preferably used as a spiral-wound membrane element. Specific preferred examples of the nanofiltration membrane element include GE Sepa, which is a cellulose acetate nanofiltration membrane manufactured by GE Osmonics; NF99 and NF99HF, which are nanofiltration membranes having a functional layer composed of a polyamide, manufactured by Alfa-Laval; NF-45, NF-90, NF-200, NF-270 and NF-400, which are nanofiltration membranes having a functional layer composed of a cross-linked piperazine polyamide, manufactured by Filmtec Corporation; and SU-210, SU-220, SU-600 and SU-610, which are nanofiltration membrane modules having a functional layer composed of a polyamide containing a cross-linked piperazine polyamide as a major component, manufactured by TORAY INDUSTRIES, INC. The nanofiltration membrane element is more preferably NF99 or NF99HF, which are nanofiltration membranes having a functional layer composed of a polyamide, manufactured by Alfa-Laval;

NF-45, NF-90, NF-200 or NF-400, which are nanofiltration membranes having a functional layer composed of a cross-linked piperazine polyamide, manufactured by Filmtec Corporation; or SU-210, SU-220, SU-600 or SU-610, which are nanofiltration membrane modules having a functional layer composed of a polyamide containing a cross-linked piperazine polyamide as a major component, manufactured by TORAY INDUSTRIES, INC. The nanofiltration membrane element is still more preferably SU-210, SU-220, SU-600 or SU-610, which are nanofiltration membrane modules having a functional layer composed of a polyamide containing a cross-linked piperazine polyamide as a major component, manufactured by TORAY INDUSTRIES, INC.

In the filtration through a nanofiltration membrane, the aqueous sugar solution obtained in Step (2) is preferably supplied to the nanofiltration membrane at a pressure within the range of 0.1 MPa to 8 MPa. In cases where the pressure is within the preferred range, the membrane permeation rate does not decrease, while there is no risk of damaging of the membrane. Further, in cases where the filtration pressure is 0.5 MPa to 6 MPa, the membrane permeation flux is high, so that the sugar solution can be allowed to permeate efficiently, and there is hardly the risk of damaging of the membrane, which is more preferred. The pressure is especially preferably 1 MPa to 4 MPa.

The sugar components contained in the refined sugar solution obtained from the feed side of the nanofiltration membrane are sugars derived from the cellulose-containing biomass, but the ratios of these sugar components are not necessarily the same as those of the sugar components obtained by the hydrolysis in Step (1), depending on the removal performance of the nanofiltration membrane. The monosaccharides contained in the refined sugar solution of the present invention comprise glucose and/or xylose as a major component(s). The ratio between glucose and xylose varies depending on the step of hydrolysis in Step (1) and on the removal performance of the nanofiltration membrane, and is not restricted in the present invention. For example, in cases where the hydrolysis was carried out mainly for hemicellulose, xylose is the major monosaccharide component, while in cases where only the cellulose component was separated after degradation of hemicellulose and subjected to hydrolysis, glucose is the major monosaccharide component. Further, in cases where degradation of hemicellulose and degradation of cellulose were carried out without separation, glucose and xylose are contained as major monosaccharide components.

Before the filtration through a nanofiltration membrane, the aqueous sugar solution may once be concentrated using a concentrator such as an evaporator, or the refined sugar solution may be further filtered through a nanofiltration membrane to increase the concentration. In view of reducing the energy for concentration, the step of further increasing the concentration by filtering the refined sugar solution through a nanofiltration membrane is preferably employed. The membrane used in this concentration step is a membrane filter that removes ions and low-molecular-weight molecules using as the driving force a pressure difference larger than the osmotic pressure of the liquid to be treated, and examples of the membrane which can be used include cellulose membranes such as those made of cellulose acetate and membranes produced by polycondensing a polyfunctional amine compound and a polyfunctional acid halide to provide a separation functional layer made of a polyamide on a microporous support membrane. In order to suppress dirt, that is, fouling, on the surface of the nanofiltration membrane, it is also preferred to employ a low-fouling membrane to be used for mainly sewage treatment, which is prepared by covering the surface of a separation functional layer made of a polyamide with an aqueous solution of a compound having at least one reactive group reactive with an acid halide group to form covalent bonds between acid halide groups remaining on the surface of the separation functional layer and the reactive group(s). Specific examples of the nanofiltration membrane to be used for the concentration are the same as those for the above-described nanofiltration membrane and reverse osmosis membrane.

A method for producing a chemical product using, as a fermentation feedstock, a concentrated aqueous sugar solution obtained by the method of the present invention for producing a concentrated aqueous sugar solution is described below.

By using a concentrated aqueous sugar solution obtained by the present invention as a fermentation feedstock, chemical products can be produced. The concentrated aqueous sugar solution obtained by the present invention contains, as a major component (s), glucose and/or xylose, which are carbon sources for growth of microorganisms and cultured cells. On the other hand, the contents of fermentation- inhibiting substances such as furan compounds, organic acids and aromatic compounds are very small. Therefore, the concentrated aqueous sugar solution can be effectively used as a fermentation feedstock, especially as a carbon source.

Examples of the microorganism or cultured cell used in the method of the present invention for producing a chemical product include yeasts such as baker's yeast, which are commonly used in the fermentation industry; bacteria such as *E. coli* and coryneform bacteria; filamentous fungi; actinomycetes; animal cells; and insect cells. The microorganism or cultured cell used may be one isolated from a natural environment, or may be one whose properties were partially modified by mutation or genetic recombination. In particular, since an aqueous sugar solution derived from a cellulose-containing biomass contains pentoses such as xylose, microorganisms having enhanced metabolic pathways for pentoses may be preferably used.

The medium to be used is preferably a liquid medium containing, in addition to the concentrated aqueous sugar solution, a nitrogen source(s), inorganic salt(s), and, as required, organic micronutrient(s) such as an amino acid(s) and/or vitamin(s). The concentrated aqueous sugar solution of the present invention contains as carbon sources monosaccharides which can be used by microorganisms, such as glucose and xylose, but, in some cases, sugars such as glucose, sucrose, fructose, galactose and lactose; saccharified starch liquids containing these sugars; sweet potato molasses; sugar beet molasses; high test molasses; organic acids such as acetic acid; alcohols such as ethanol; glycerin; and the like may be further added as carbon sources, to use the concentrated aqueous sugar solution as a fermentation feedstock. Examples of the nitrogen sources used include ammonia gas, aqueous ammonia, ammonium salts, urea and nitric acid salts; and other organic nitrogen sources used supplementarily such as oilcakes, soybean-hydrolyzed liquids, casein digests, other amino acids, vitamins, corn steep liquors, yeasts or yeast extracts, meat extracts, peptides such as peptones, and cells of various fermentation microorganisms and hydrolysates thereof. Examples of the inorganic salts which may be added as appropriate include phosphoric acid salts, magnesium salts, calcium salts, iron salts and manganese salts.

In cases where the microorganism requires particular nutrients for its growth, the nutrients may be added as preparations or natural products containing these. An anti-forming agent may also be used as required.

Culturing of the microorganism is usually carried out at a pH within the range of 4 to 8, at a temperature within the range of 20 to 40°C. The pH of the culture medium is adjusted in advance with an inorganic or organic acid, alkaline substance, urea, calcium carbonate, ammonia gas or the like to a predetermined pH within the range of, usually, 4 to 8. In cases where the feed rate of oxygen needs to be increased, a method can be employed in which, for example, the oxygen concentration is maintained at not less than 21 % by adding oxygen into the air; the culturing is carried out under pressure; the stirring rate is increased; or the ventilation volume is increased.

As the method for producing a chemical product using, as a fermentation feedstock, a concentrated aqueous sugar solution obtained by the method of the present invention for producing a concentrated aqueous sugar solution, a fermentation culture method known to those skilled in the art may be employed, and, in view of productivity, the continuous culture method disclosed in WO2007/097260 is preferably employed.

The chemical product produced is not restricted as long as it is a substance produced in the culture medium by the above microorganism or cell. Specific examples of the chemical product produced include alcohols, organic acids, amino acids and nucleic acids, which are substances mass- produced in the fermentation industry. Examples the substances include alcohols such as ethanol, 1, 3- propanediol, 1, 4- propanediol and glycerol; organic acids such as acetic acid, lactic acid, pyruvic acid, succinic acid, malic acid, itaconic acid and citric acid; nucleic acids such as nucleosides including inosine and guanosine, and nucleotides including inosinic acid and guanylic acid; and diamine compounds such as cadaverine. Further, the concentrated aqueous sugar solution obtained by the method of the present invention for producing a refined sugar solution may also be applied to production of substances such as enzymes, antibiotics and recombinant proteins.

An apparatus for production of the concentrated aqueous sugar solution used in the method of the present invention for producing a concentrated aqueous sugar solution is described below with reference to a drawing.

Fig. 1 is a schematic flow chart showing an embodiment of the present invention. In this embodiment, Procedure B-a method wherein acid treatment is carried out followed by use of an enzyme-was employed as an example of the step of hydrolysis of a cellulose-containing biomass. In Fig. 1, the acid treatment tank 1 is an acid treatment tank for hydrolysis of a biomass with an acid; the biomass storage tank 2 is a storage tank for the biomass treated with the acid; the aqueous enzyme solution storage tank 3 is a storage tank for an aqueous enzyme solution; the enzymatic saccharification tank 4 is an enzymatic saccharification tank for hydrolysis of the biomass with the enzyme; the first pump 5 is a pump that produces a pressure of about 0.5 MPa, for supplying a saccharified liquid to a microfiltration membrane and/or ultrafiltration membrane; the MF/UF membrane 6 is a microfiltration membrane and/or ultrafiltration membrane; the aqueous sugar solution storage tank 7 is a storage tank for the aqueous sugar solution recovered from the permeate side of the microfiltration membrane and/or ultrafiltration membrane; the second pump 8 is a high-pressure pump that can produce a pressure of about 1 to 8 MPa, for supplying the saccharified liquid to a reverse osmosis membrane; the RO membrane 9 is a reverse osmosis membrane; the third pump 10 is a backwashing pump for the microfiltration membrane and/or ultrafiltration membrane; the fourth pump 11 is a pump for injection of an agent; the agent tank 12 is an agent tank for storing an agent for washing the microfiltration membrane and/or ultrafiltration membrane; the reuse water tank 13 is a reuse water tank for storing at least a part of the permeate from the reverse osmosis membrane; and the fifth pump 14 is a pump as a means for returning at least a part of the permeate from the reverse osmosis membrane to the respective steps.

$V_1$, $V_2$, $V_3$, $V_4$, $V_5$, $V_6$, $V_7$, $V_8$, $V_9$, $V_{10}$, $V_{11}$, $V_{12}$, $V_{13}$, $V_{14}$ and $V_{15}$ represent valves, and opening and closing each of $V_{10}$, $V_{11}$, $V_{12}$ and $V_{13}$ enable switching of the step to which the reuse water as at least a part of the permeate from the reverse osmosis membrane should be returned. In cases where the reuse water is not used for backwashing of the microfiltration membrane and/or ultrafiltration membrane, the means for returning at least a part of the permeate from the reverse osmosis membrane to the respective steps may be one requiring no power or less power, such as sending of the liquid by utilization of the hydraulic head difference instead of the fifth pump 14.

The washing time for the microfiltration membrane and/or ultrafiltration membrane is not restricted, and is preferably within the range of 1 to 180 seconds, especially preferably 30 to 120 seconds. In cases where the washing

time is within the preferred range, a sufficient washing effect can be obtained, while the operation time of the microfiltration membrane and/or ultrafiltration membrane can be sufficiently secured. The washing flux is not restricted, and is preferably within the range of 0.1 to 10 $m^3/m^2$/day. In cases where the washing flux is within the preferred range, the biomass residue and the like accumulated or attached on the membrane surface or inside the membrane can be sufficiently removed, while no load is imposed on the microfiltration membrane and/or ultrafiltration membrane.

Further, when the microfiltration membrane and/or ultrafiltration membrane is/are washed using at least a part of the permeate from the reverse osmosis membrane, it is also preferred to send a gas into the primary side of the microfiltration membrane and/or ultrafiltration membrane to vibrate the microfiltration membrane and/or ultrafiltration membrane.

The frequency of washing of the microfiltration membrane and/or ultrafiltration membrane using at least a part of the permeate from the reverse osmosis membrane is not restricted, and the washing is preferably performed within the range of 1 to 200 times per day. In cases where the washing frequency is within the preferred range, the effect of saving water by recovery and reuse of the permeate from the reverse osmosis membrane can be sufficiently produced, while the operation time of the microfiltration membrane and/or ultrafiltration membrane can be sufficiently secured.

EXAMPLES

The method of the present invention for producing a concentrated aqueous sugar solution will now be described in more detail by way of Examples. However, the present invention is not limited to these Examples.

(Method for Analyzing Monosaccharide Concentrations)

The concentrations of monosaccharides (glucose and xylose) contained in the obtained aqueous sugar solution were quantified under the HPLC conditions described below, based on comparison with standard samples.

Column: Luna $NH_2$ (manufactured by Phenomenex, Inc.)
Mobile phase: ultrapure water:acetonitrile=25:75 (flow rate, 0.6 mL/min.)
Reaction solution: none
Detection method: RI (differential refractive index)
Temperature: 30°C

(Method for Analyzing Enzyme Concentration)

The protein concentration was measured based on the assumption that all the protein components contained in the liquid are enzymes. The protein concentration was colorimetrically measured using BCA measurement kit (BCA Protein Assay Regent kit, PIERCE) by measurement of absorbance at 562 nm using bovine serum albumin (2 mg/mL) as a standard sample.

(Example 1)

Step (1), which is the step of hydrolyzing a cellulose-containing biomass, wherein 0.1 to 15% by weight of dilute sulfuric acid and enzymes are used is described below.

As a cellulose-containing biomass, about 800 g of rice straw was used. The cellulose-containing biomass was immersed in 2% aqueous sulfuric acid solution (5,880 g of water and 120 g of concentrated sulfuric acid), and subjected to treatment using an autoclave (manufactured by Nitto Koatsu Co., Ltd.) at 150°C for 30 minutes. After the treatment, solid-liquid separation was carried out to separate sulfuric acid-treated cellulose from the aqueous sulfuric acid solution (hereinafter referred to as "dilute-sulfuric-acid treatment liquid"). Subsequently, the sulfuric acid-treated cellulose was mixed with the dilute-sulfuric-acid treatment liquid with stirring such that the solids concentration is about 12% by weight, and the pH was adjusted to about 5 with sodium hydroxide, to obtain a mixture. This mixture was dried to measure the water content. As a result, the mixture was found to contain 5,580 g of water and 750 g of a cellulose-containing biomass.

Subsequently, an enzyme containing, as cellulases, a total of 50 g of *Trichoderma* cellulase (Sigma Aldrich Japan) and Novozyme 188 (*Aspergillus niger*- derived β- glucosidase preparation, Sigma Aldrich Japan) was dissolved in 450 g of water, to prepare 500 g of an aqueous enzyme solution. To the above mixture, 500 g of this aqueous enzyme solution was added, and the resulting mixture was subjected to hydrolysis reaction at 50°C for 3 days with stirring, to obtain an aqueous sugar solution. In order to analyze monosaccharide concentrations and fermentation- inhibiting substance concentrations in the obtained aqueous sugar solution, the solution was centrifuged at 3, 000 G to perform solid- liquid separation. As a result of the analysis, the aqueous sugar solution was found to contain 241 g of glucose

and 119 g of xylose as monosaccharides, and 8.5 g of furfural and 520 mg of vanillin as fermentation- inhibiting substances. Further, as a result of measurement of the water content by drying the aqueous sugar solution, the solution was found to contain 6, 030 g of water.

In Step (2), the aqueous sugar solution obtained in Step (1) was supplied to a microfiltration membrane at a pressure of 100 kPa at a temperature of 25°C to perform cross-flow filtration, and the aqueous sugar solution was recovered from the permeate side. The linear velocity on the membrane surface during the cross-flow filtration was kept at 30 cm/sec. In terms of the microfiltration membrane, the hollow fiber membrane made of polyvinylidene fluoride having a nominal pore size of 0.05 μm used in a microfiltration membrane module manufactured by TORAY INDUSTRIES, INC., "TORAYFIL" (registered trademark) HFS, was cut out to prepare a miniature module composed of 50 hollow fiber membranes having a length of 200 mm, and the prepared module was used for filtration. As a result of analysis of monosaccharide concentrations and fermentation-inhibiting substance concentrations in the obtained aqueous sugar solution, the aqueous sugar solution was found to contain 228 g of glucose and 113 g of xylose as monosaccharides, and 8.0 g of furfural and 820 mg of vanillin as fermentation-inhibiting substances. Further, as a result of measurement of the water content by drying the aqueous sugar solution, the solution was found to contain 5,870 g of water.

As the washing liquid for backwashing of the microfiltration membrane, 12,000 g of clean water was used.

In Step (3), the aqueous sugar solution obtained in Step (2) was supplied to a nanofiltration membrane at a pressure of 3 MPa at a temperature of 25°C to perform cross-flow filtration. While the concentrated aqueous sugar solution was recovered from the feed side, the permeate was recovered from the permeate side, to obtain a concentrated aqueous sugar solution and a permeate from the nanofiltration membrane. The linear velocity on the membrane surface during the cross-flow filtration was kept at 30 cm/sec. In terms of the nanofiltration membrane, the polyamide nanofiltration membrane used in the polyamide nanofiltration membrane module "SU-600" manufactured by TORAY INDUSTRIES, INC. was cut out and used. When the polyamide nanofiltration membrane used in "SU-600" was subjected to measurement using 500 mg/L saline at 0.34 MPa, 25°C and pH 6.5, the salt rejection rate was 55%, and the permeation flow rate per unit membrane area was 0.7 $m^3/m^2$/day. As a result of analysis of monosaccharide concentrations and fermentation-inhibiting substance concentrations in the obtained concentrated aqueous sugar solution, the refined sugar solution was found to contain 222 g of glucose and 101 g of xylose as monosaccharides, and 1.6 g of furfural and 405 mg of vanillin as fermentation-inhibiting substances. Further, as a result of measurement of the water content by drying the aqueous sugar solution, the solution was found to contain 3,740 g of water.

On the other hand, as a result of analysis of monosaccharide concentrations and fermentation-inhibiting substance concentrations in the permeate obtained from the nanofiltration membrane, the permeate from the nanofiltration membrane was found to contain 6 g of glucose and 12 g of xylose as monosaccharides, and 6.4 g of furfural and 415 mg of vanillin as fermentation-inhibiting substances. Further, as a result of measurement of the water content by drying the permeate obtained from the nanofiltration membrane, the permeate was found to contain 2,130 g of water.

The whole amount of the permeate obtained from the nanofiltration membrane was mixed with 9,900 g of clean water, and the resulting mixture was used as the washing liquid for backwashing of the microfiltration membrane in Step (2). As a result, 2,100 g of clean water could be saved.

In Step (4), the refined sugar solution obtained in Step (3) was supplied to a reverse osmosis membrane at a pressure of 3 MPa at a temperature of 25°C to perform cross-flow filtration. While the concentrated aqueous sugar solution was recovered from the feed side, the permeate was recovered from the permeate side, to obtain a concentrated aqueous sugar solution and a permeate from the reverse osmosis membrane. The linear velocity on the membrane surface during the cross-flow filtration was kept at 30 cm/sec. In terms of the reverse osmosis membrane, the polyamide reverse osmosis membrane used in the polyamide reverse osmosis membrane module "TMG10" manufactured by TORAY INDUSTRIES, INC. was cut out and used. When the polyamide reverse osmosis membrane used in "TMG10" was subjected to measurement using 500 mg/L saline at 0.76 MPa, 25°C and pH 6.5, the salt rejection rate was 99.5%, and the permeation flow rate per unit membrane area was 1.0 $m^3/m^2$/day. As a result of analysis of monosaccharide concentrations and fermentation-inhibiting substance concentrations in the obtained concentrated aqueous sugar solution, the concentrated aqueous sugar solution was found to contain 220 g of glucose and 100 g of xylose as monosaccharides, and 1.5 g of furfural and 400 mg of vanillin as fermentation-inhibiting substances. Further, as a result of measurement of the water content by drying the aqueous sugar solution, the solution was found to contain 1,600 g of water.

On the other hand, as a result of analysis of monosaccharide concentrations and fermentation-inhibiting substance concentrations in the permeate obtained from the reverse osmosis membrane, the permeate from the reverse osmosis membrane was found to contain 2 g of glucose and 1 g of xylose as monosaccharides, and 0.1 g of furfural and 5 mg of vanillin as fermentation-inhibiting substances. Further, as a result of measurement of the water content by drying the permeate obtained from the reverse osmosis membrane, the permeate was found to contain 2,140 g of water.

Since the permeate obtained from the reverse osmosis membrane was clear with a water content of not less than 99%, it was reused as 450 g of water for preparing 500 g of the aqueous enzyme solution in the step of hydrolysis of a cellulose-containing biomass in Step (1). The remaining 1,690 g of water was reused as water for 2% aqueous sulfuric acid solution.

(Reference Example 1) Method for Measuring Cellulase Activity

The cellulase activity was measured and evaluated by the following procedures in terms of four types of degradation activities: a) Avicel- degrading activity; b) carboxymethyl cellulose (CMC)- degrading activity; c) cellobiose-degrading activity; and d) xylan- degrading activity.

a) Avicel-degrading Activity

To an enzyme liquid (prepared under predetermined conditions), Avicel (manufactured by Merck) was added at 1 g/L and sodium acetate buffer (pH 5.0) was added at 100 mM, followed by allowing the resulting mixture to react at 50°C for 24 hours. This reaction liquid was prepared in a 1-mL tube, and the reaction was allowed to proceed with mixing by rotation under the above-described conditions. Thereafter, the tube was subjected to centrifugation, and the glucose concentration in the supernatant component was measured. The measurement of the glucose concentration was carried out according to the method described in Reference Example 3. The concentration of the produced glucose (g/L) was used as it is as the activity value of the Avicel-degrading activity.

b) CMC-degrading Activity

To an enzyme liquid, carboxymethyl cellulose was added at 10 g/L and sodium acetate buffer (pH 5.0) was added at 100 mM, followed by allowing the resulting mixture to react at 50°C for 0.5 hour. This reaction liquid was prepared in a 1-mL tube, and the reaction was allowed to proceed with mixing by rotation under the above-described conditions. Thereafter, the tube was subjected to centrifugation, and the glucose concentration in the supernatant component was measured. The measurement of the glucose concentration was carried out according to the method described in Reference Example 3. The concentration of the produced glucose (g/L) was used as it is as the activity value of the CMC-degrading activity.

c) Cellobiose-degrading Activity

To an enzyme liquid, cellobiose (Wako Pure Chemical Industries, Ltd.) was added at 500 mg/L and sodium acetate buffer (pH 5.0) was added at 100 mM, followed by allowing the resulting mixture to react at 50°C for 0.5 hour. This reaction liquid was prepared in a 1-mL tube, and the reaction was allowed to proceed with mixing by rotation under the above-described conditions. Thereafter, the tube was subjected to centrifugation, and the glucose concentration in the supernatant component was measured. The measurement of the glucose concentration was carried out according to the method described in Reference Example 3. The concentration of the produced glucose (g/L) was used as it is as the activity value of the cellobiose-degrading activity.

d) Xylan-degrading Activity

To an enzyme liquid, xylan (Birch wood xylan, Wako Pure Chemical Industries, Ltd.) was added at 10 g/L and sodium acetate buffer (pH 5.0) was added at 100 mM, followed by allowing the resulting mixture to react at 50°C for 4 hours. This reaction liquid was prepared in a 1-mL tube, and the reaction was allowed to proceed with mixing by rotation under the above-described conditions. Thereafter, the tube was subjected to centrifugation, and the xylose concentration in the supernatant component was measured. The measurement of the xylose concentration was carried out according to the method described in Reference Example 3. The concentration of the produced xylose (g/L) was used as it is as the activity value of the xylose-degrading activity.

(Reference Example 2) Preparation of Permeate from Reverse Osmosis Membrane Derived from Step (3)

Although the reverse osmosis membrane permeates 1 to 8 used in Examples 2 and 3 and Comparative Examples 1 and 2 were prepared by the same procedure, the lot of the raw material rice straw and the date of preparation were different among these. The procedure for the preparation was as follows.

In Step (1), 2,940 g of the reverse osmosis membrane permeate obtained in Step (3) and 60 g of concentrated sulfuric acid were added to about 400 g of rice straw as a cellulose-containing biomass, and the resulting mixture was suspended, followed by treating the resulting suspension in an autoclave (manufactured by Nitto Koatsu Co., Ltd.) at 150°C for 30 minutes. Thereafter, a mixture was obtained by adjusting the pH to about 5 with sodium hydroxide.

Subsequently, an enzyme containing, as cellulases, a total of 25 g of *Trichoderma* cellulase (Sigma Aldrich Japan) and Novozyme 188 (*Aspergillus niger*- derived β- glucosidase preparation, Sigma Aldrich Japan) was dissolved in 225 g of water, to prepare 250 g of an aqueous enzyme solution. To the above mixture, 250 g of this aqueous enzyme

solution was added, and the resulting mixture was subjected to hydrolysis reaction at 50°C for 3 days with stirring, to obtain an aqueous sugar solution.

Subsequently, in Step (2), the aqueous sugar solution obtained in Step (1) was supplied to a microfiltration membrane at a pressure of 100 kPa at a temperature of 25°C to perform cross- flow filtration, and the aqueous sugar solution was recovered from the permeate side. The linear velocity on the membrane surface during the cross- flow filtration was kept at 30 cm/sec. In terms of the microfiltration membrane, the hollow fiber membrane made of polyvinylidene fluoride having a nominal pore size of 0.05 μm used in a microfiltration membrane module manufactured by TORAY INDUSTRIES, INC., "TORAYFIL" (registered trademark) HFS, was cut out to prepare a miniature module composed of 22 hollow fiber membranes having an internal diameter of 10 mm and a length of 200 mm, and the prepared module was used for filtration.

Subsequently, in Step (3), the concentrated aqueous sugar solution obtained in Step (2) was supplied to a reverse osmosis membrane at a pressure of 3 MPa at a temperature of 25°C to perform cross- flow filtration, and the concentrated aqueous sugar solution was recovered from the feed side. The linear velocity on the membrane surface during the cross- flow filtration was kept at 30 cm/sec. In terms of the reverse osmosis membrane, the polyamide reverse osmosis membrane used in the polyamide reverse osmosis membrane module "TMG10" manufactured by TORAY INDUSTRIES, INC. was cut out and used. When the polyamide reverse osmosis membrane used in "TMG10" was subjected to measurement using 500 mg/L saline at 0.76 MPa, 25°C and pH 6.5, the salt rejection rate was 99.5%, and the permeation flow rate per unit membrane area was 1.0 $m^3/m^2$/day. The obtained permeate was provided as the reverse osmosis membrane permeates 1 to 8 for Examples 2 and 3 and Comparative Examples 1 and 2.

(Example 2)

An enzyme was diluted using the reverse osmosis membrane permeates 1 and 2 described in Table 1, which were obtained in Step (3) according to Reference Example 2 and whose acetic acid concentrations were less than 1.5 g/L. The enzyme activity of each obtained enzyme dilution was measured according to Reference Example 1. As the enzyme, Accellerase DUET manufactured by Genencor was used. For comparison, the degradation activity obtained by use of ultrapure water for diluting the enzyme was defined as 1, and each enzyme activity was represented as a relative value with respect to this standard.

(Comparative Example 1)

An enzyme was diluted using the reverse osmosis membrane permeates 3 and 4 described in Table 1, which were obtained in Step (3) according to Reference Example 2 and whose acetic acid concentrations were not less than 1.5 g/L. The enzyme activity of each obtained enzyme dilution was measured according to Reference Example 1. As the enzyme, Accellerase DUET manufactured by Genencor was used. For comparison, the degradation activity obtained by use of ultrapure water for diluting the enzyme was defined as 1, and each enzyme activity was represented as a relative value with respect to this standard.

[Table 1]

| | Blank | Example 2 | | Comparative Example 1 | |
|---|---|---|---|---|---|
| | ultrapure water | Reverse osmosis membrane permeated water 1 | Reverse osmosis membrane permeated water 2 | Reverse osmosis membrane permeated water 3 | Reverse osmosis membrane permeated water 4 |
| Acetic acid concentration | 0 g/L | 1.4 g/L | 1.3 g/L | 1.7 g/L | 1.9 g/L |
| Avicel activity | 1.00 | 0.99 | 1.00 | 0.80 | 0.80 |
| glucosidase activity | 1.00 | 1.00 | 0.98 | 0.82 | 0.80 |
| Xylan decomposition activity | 1.00 | 1.00 | 1.00 | 0.78 | 0.79 |

From the results of Example 2 and Comparative Example 1, it was proved that a decrease in the enzyme activity can be suppressed by using a processing water with an acetic acid concentration of less than 1.5 g/L as an enzyme-diluting water.

(Reference Example 3) Ethanol Fermentation by Yeast

In Comparative Example 2 and Example 3, ethanol fermentation was carried out using the *Saccharomyces cerevisiae* OC2 strain (wine yeast) as follows, and the obtained sugar liquids were evaluated.
Fermentation media were prepared with the composition below using the aqueous sugar solutions obtained in Comparative Example 2 and Example 3, and subjected to filter sterilization (Millipore Stericup 0.22 μm, Merck) before being used for fermentation.

<Fermentation medium>

| | |
|---|---|
| Glucose | 30 g/L, final concentration |
| Synthetic Complete Dropout Mix | 3.8g/L |
| Yeast Nitrogen Base | 1.7g/L |

The glucose concentration was measured using Glucose Test Wako (manufactured by Wako Pure Chemical Industries, Ltd.). The amount of ethanol produced in each culture was measured by gas chromatography (Shimadzu GC-2010 capillary GC TC-1 (GL science) 15 meter L. × 0.53 mm I.D., df 1.5 μm) using a hydrogen flame ionization detector.
The OC2 strain was cultured with shaking in 5 mL of the fermentation medium overnight (preculture). Subsequently, the preculture was inoculated to 100 mL of the fermentation medium, and culture was performed in a 500-mL Sakaguchi flask for 24 hours with shaking (main culture), followed by evaluation of the ethanol production at Hour 24.

(Reference Example 4) Method for Preparing Sugar Liquid

In Step (1), 2,940 g of the reverse osmosis membrane permeate obtained in Step (3) and 60 g of concentrated sulfuric acid were added to about 430 g of rice straw as a cellulose-containing biomass. The resulting mixture was suspended and subjected to treatment using an autoclave (manufactured by Nitto Koatsu Co., Ltd.) at 150°C for 30 minutes. Thereafter, a mixture was obtained by adjusting the pH to about 5 with sodium hydroxide.
Subsequently, an enzyme containing, as cellulases, a total of 25 g of *Trichoderma* cellulase (Sigma Aldrich Japan) and Novozyme 188 (*Aspergillus niger*- derived β- glucosidase preparation, Sigma Aldrich Japan) was dissolved in 225 g of water, to prepare 250 g of an aqueous enzyme solution. To the above mixture, 250 g of this aqueous enzyme solution was added, and the resulting mixture was subjected to hydrolysis reaction at 50°C for 3 days with stirring, to obtain an aqueous sugar solution.
Subsequently, in Step (2), the aqueous sugar solution obtained in Step (1) was supplied to a microfiltration membrane at a pressure of 100 kPa at a temperature of 25°C to perform cross- flow filtration, and the aqueous sugar solution was recovered from the permeate side. The linear velocity on the membrane surface during the cross- flow filtration was kept at 30 cm/sec. In terms of the microfiltration membrane, the hollow fiber membrane made of polyvinylidene fluoride having a nominal pore size of 0.05 μm used in a microfiltration membrane module manufactured by TORAY INDUSTRIES, INC., "TORAYFIL" (registered trademark) HFS, was cut out to prepare a miniature module composed of 22 hollow fiber membranes having an internal diameter of 10 mm and a length of 200 mm, and the prepared module was used for filtration.
Subsequently, in Step (3), the concentrated aqueous sugar solution obtained in Step (2) was supplied to a reverse osmosis membrane at a pressure of 3 MPa at a temperature of 25°C to perform cross- flow filtration, and the concentrated aqueous sugar solution was recovered from the feed side. The linear velocity on the membrane surface during the cross- flow filtration was kept at 30 cm/sec. In terms of the reverse osmosis membrane, the polyamide reverse osmosis membrane used in the polyamide reverse osmosis membrane module "TMG10" manufactured by TORAY INDUSTRIES, INC. was cut out and used. When the polyamide reverse osmosis membrane used in "TMG10" was subjected to measurement using 500 mg/L saline at 0.76 MPa, 25°C and pH 6.5, the salt rejection rate was 99.5%, and the permeation flow rate per unit membrane area was 1.0 $m^3/m^2$/day.

(Example 3)

As the water for suspending rice straw in Step (1), the reverse osmosis membrane permeates 5 and 6 described in Table 2 with acetic acid concentrations of less than 1.5 g/L, which were obtained in Step (3) according to Reference Example 2, were used to obtain concentrated aqueous sugar solutions according to the method in Reference Example 4.

Using each obtained aqueous sugar solution as a glucose source, a fermentation medium was prepared, and preculture and main culture were performed as described in Reference Example 3 to perform ethanol fermentation. In the preculture, reagent monosaccharides were used, and the aqueous sugar solution was used only in the main culture. The glucose consumption and the concentration of accumulated ethanol after the ethanol fermentation are also shown in Table 2.

[Table 2]

| | Example 3 | | Comparative Example 2 | |
|---|---|---|---|---|
| | Reverse osmosis membrane permeated water 5 | Reverse osmosis membrane permeated water 6 | Reverse osmosis membrane permeated water 7 | Reverse osmosis membrane permeated water 8 |
| Acetic acid concentration | 1.2 g/L | 1.4 g/L | 1.8 g/L | 1.9 g/L |
| Glucose comsumption | 27 g/L | 25 g/L | 16 g/L | 14 g/L |
| Accumulated ethanol concentration | 11.3 g/L | 10.3 g/L | 6.1 g/L | 5.0 g/L |

(Comparative Example 2)

As the water for suspending rice straw in Step (1), the reverse osmosis membrane permeates 7 and 8 described in Table 2 with acetic acid concentrations of not less than 1.5 g/L, which were obtained in Step (3) according to Reference Example 2, were used to obtain concentrated aqueous sugar solutions according to the method in Reference Example 4.

Using each obtained aqueous sugar solution as a glucose source, a fermentation medium was prepared, and preculture and main culture were performed as described in Reference Example 3 to perform ethanol fermentation. In the preculture, reagent monosaccharides were used, and the aqueous sugar solution was used only in the main culture. The glucose consumption and the concentration of accumulated ethanol after the ethanol fermentation are also shown in Table 2.

As is apparent from the results of Example 3 and Comparative Example 2, ethanol fermentation could be carried out without inhibition when the reverse osmosis membrane permeates with acetic acid concentrations of less than 1.5 g/L, which were obtained in Step (3), were used as the water for suspending rice straw (biomass).

INDUSTRIAL APPLICABILITY

Since the present invention provides a method comprising hydrolyzing a cellulose-containing biomass to produce an aqueous sugar solution, treating the aqueous sugar solution with a microfiltration membrane and/or an ultrafiltration membrane to remove the biomass residue, and then concentrating the aqueous sugar solution by treatment with a reverse osmosis membrane to increase the sugar concentration, wherein the permeate discarded from the reverse osmosis membrane is recovered and reused, water saving in the whole process can be achieved. Therefore, the object, construction of an environment-conscious society, can be achieved while the cost of fermentation production of various chemical products using the concentrated aqueous sugar solution as a fermentation feedstock can be reduced.

DESCRIPTION OF SYMBOLS

1 Acid treatment tank
2 Biomass storage tank

3 Aqueous enzyme solution storage tank
4 Enzymatic saccharification tank
5 First pump
6 MF/UF membrane
7 Aqueous sugar solution storage tank
8 Second pump
9 RO membrane
10 Third pump
11 Fourth pump
12 Agent tank
13 Reuse water tank
14 Fifth pump

## Claims

**1.** A method for producing a concentrated aqueous sugar solution using a cellulose-containing biomass as a raw material, said method comprising the steps of:

(1) hydrolyzing a cellulose-containing biomass to produce an aqueous sugar solution;
(2) filtering said aqueous sugar solution obtained in (1) through a microfiltration membrane and/or an ultrafiltration membrane, and recovering an aqueous sugar solution from the permeate side; and
(3) filtering said aqueous sugar solution obtained in (2) through a reverse osmosis membrane, and recovering a permeate from the permeate side and recovering a concentrated aqueous sugar solution from the feed side;
wherein at least a part of said permeate from said reverse osmosis membrane is used as at least one of a hydrothermal treatment liquid, biomass-suspending liquid, washing liquid, enzyme-diluting liquid, acid-diluting liquid and alkali-diluting liquid in said Step (1).

**2.** The method for producing a concentrated aqueous sugar solution according to claim 1, wherein, in cases where the acetic acid concentration in said permeate from said reverse osmosis membrane is less than 1.5 g/L, said permeate is used as at least one of a hydrothermal treatment liquid, enzyme-diluting liquid, acid-diluting liquid and alkali-diluting liquid in said Step (1), while in cases where said acetic acid concentration is not less than 1.5 g/L, said permeate is used as a hydrothermal treatment liquid and/or washing liquid in said Step (1).

**3.** The method for producing a concentrated aqueous sugar solution according to claim 1 or 2, wherein said reverse osmosis membrane is a composite membrane comprising polyamide as a functional layer.

**4.** The method for producing a concentrated aqueous sugar solution according to any one of claims 1 to 3, wherein said reverse osmosis membrane has a salt rejection rate of not less than 90% when measurement is carried out using 500 mg/L saline at 0.76 MPa, 25°C and pH 6.5.

**5.** The method for producing a concentrated aqueous sugar solution according to any one of claims 1 to 4, wherein said microfiltration membrane and/or ultrafiltration membrane is/are a hollow fiber membrane(s).

**6.** The method for producing a concentrated aqueous sugar solution according to any one of claims 1 to 5, said method further comprising the step of filtering said aqueous sugar solution obtained in said Step (2) through a nanofiltration membrane.

**7.** A method for producing ethanol using a yeast from a concentrated aqueous sugar solution obtained by the method according to any one of claims 1 to 6.

FIGURES

[Figure 1]

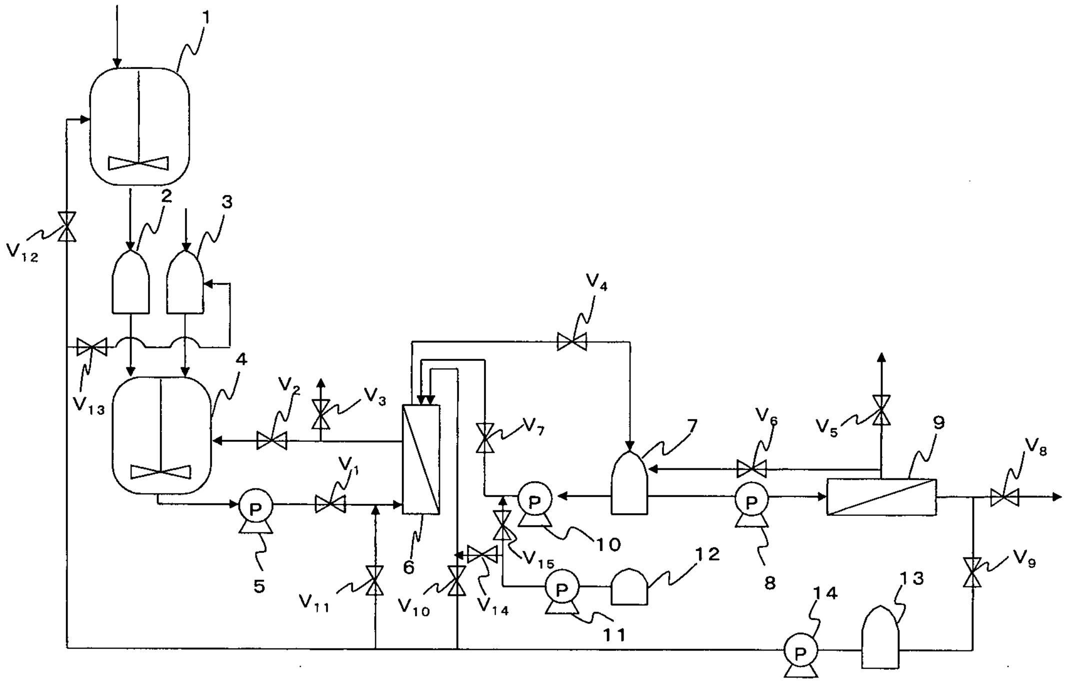

**INTERNATIONAL SEARCH REPORT**

International application No.
PCT/JP2011/078248

A. CLASSIFICATION OF SUBJECT MATTER
*C13K1/04*(2006.01)i, *B01D61/02*(2006.01)i, *B01D61/14*(2006.01)i, *B01D61/58*(2006.01)i, *B01D65/02*(2006.01)i, *B01D69/08*(2006.01)i, *B01D69/12*(2006.01)i, *B01D71/56*(2006.01)i, *B09B3/00*(2006.01)i

According to International Patent Classification (IPC) or to both national classification and IPC

B. FIELDS SEARCHED

Minimum documentation searched (classification system followed by classification symbols)
C13K1/04, B01D61/02, B01D61/14, B01D61/58, B01D65/02, B01D69/08, B01D69/12, B01D71/56, B09B3/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
CA/BIOSIS/MEDLINE/WPIDS(STN), JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| X | WO 2010/067785 A1 (Toray Industries, Inc.), 17 June 2010 (17.06.2010), entire text; particularly, paragraph [0115] (Family: none) | 1-7 |
| Y | Yutaka ADO, "Alcohol production from cellulosic biomass", Journal of Wood Science, 1989, vol.35, pages 1067 to 1072 | 1-7 |
| Y | FURUICHI M.et al., Studies on enzymic saccharification, enzyme recovery by UF and sugar concentration by RO membranes, WORLD CONGRESS 3 of CHEMICAL ENGINEERING, 1986, p.255-257 | 1-7 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 February, 2012 (01.02.12) | 14 February, 2012 (14.02.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No. PCT/JP2011/078248

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2003-219900 A (Kobe Steel, Ltd.), 05 August 2003 (05.08.2003), entire text; particularly, paragraph [0030] (Family: none) | 1-7 |
| P,X | JP 2011-223975 A (Toray Industries, Inc.), 10 November 2011 (10.11.2011), entire text; particularly, paragraph [0042] (Family: none) | 1-7 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- JP 2005229821 A
- JP 2003212888 A
- JP 2001095597 A
- JP 3041380 B
- WO 2010067785 A
- JP 2008161125 A
- JP 2008535664 A
- JP 62201606 A
- WO 2007097260 A

### Non-patent literature cited in the description

- **A. ADEN et al.** Lignocellulosic Biomass to Ethanol Process Design and Economics Utilizing Co-Current Dilute Acid Prehydrolysis and Enzymatic Hydrolysis for Corn Stover. *NREL Technical Report,* 2002
- Membrane Experiment Series, Vol. III, Artificial Membrane. Kyoritsu Shuppan Co., Ltd, 1993, vol. III, 92